# EUROPEAN PATENT SPECIFICATION

(11) **EP 3 797 739 B1**
(45) Date of publication and mention of the grant of the patent: **29.09.2021**
(21) Application number: 19863983.3
(22) Date of filing: 27.11.2019
(51) Int. Cl.: A61F 5/445, A61F 5/448

(54) **CONVEX SKIN BARRIER**
KONVEXE HAUTBARRIERE
BARRIÈRE CUTANÉE CONVEXE

(30) Priority: 31.07.2019 CN 201910705312
(43) Date of publication of application: 31.03.2021
(73) Proprietor: ZHENDE MEDICAL CO., LTD., Shaoxing, Zhejiang 312000 (CN)
(72) Inventor: CHE, Haozhao, Shaoxing, Zhejiang 312000 (CN); LU, Jianguo, Shaoxing, Zhejiang 312000 (CN); ZHANG, Xuan, Shaoxing, Zhejiang 312000 (CN)
(74) Representative: Loo, Chi Ching
(86) International application number: PCT/CN2019/121093
(87) International publication number: WO 2021/017343

(56) References cited:
- EP-A2- 0 317 326
- CN-A- 108 852 604
- CN-A- 110 279 506
- CN-U- 206 120 534
- CN-Y- 201 005 837
- CN-Y- 201 179 135
- US-A- 5 429 626

## Description

### Technical Field

The invention relates to a field of medical products, in particular to a convex stoma chassis.

### Background Art

The International Consensus on the Use of Convex Surfaces published in 2017 clearly points out that convex chassis ostomy bags can be used for patients with intestinal ostomy, and the convex chassis can fit more closely with the skin around the stoma to reduce the risk of leakage. Domestic nursing workers have found that the time for replacing ostomy bags is significantly enlarged after a convex chassis treatment for patients with intestinal fistula and digestive tract ostomy compared with flat chassis ostomy bags, which greatly reduces the nursing workload and medical expenses. At the same time, the stimulation of stoma discharge to surrounding skins is reduced, which is beneficial to a regression of inflammation, accelerates a healing of the damaged skin, and shortens a treatment time of anastomosis.

Generally, the clinical application scope of convex chassis ostomy products includes the following cases: the case when the stoma structure/height is not ideal; the case when the abdominal wall around the stoma is not ideal; the case when conditions of the skin around the stoma is not ideal, and in fact, there are also some special clinical situations that require the use of convex surfaces, including: parastomal fistula, unexplained leakage, severe early postoperative leakage, high excretion stoma, urinary tract stoma, etc. Document EP 0 317 326 discloses an ostomy bag comprising a convex ostomy plate with a plastic disc and a plastic part fixed to it. A foam ring is secured to the plate with a hotmelt adhesive. The bag is equipped with a hydrocolloid skin barrier ring.

For the design of the convex stoma chassis on the market, excrements are easy to leak and contact with a hot melt adhesive, thus eroding the hot melt adhesive, shortening the service life of the chassis, and thus the market needs a convex stoma chassis capable of completely avoiding excrement leakage which is solved by the invention.

### Summary of the Invention

In order to solve the defects of the prior art, the invention aims to provide a convex stoma chassis, in which a welding ring is disposed between a plastic part and a backing, so that the leakage of the excrement and the corrosion of the hot melt adhesive can be completely avoided, and thus improving the use stability and the service life.

In order to achieve the above object, the invention adopts the following technical scheme:
A convex stoma chassis comprises a rubber disc, a plastic part fixed on the rubber disc, and a hot melt adhesive connected between the rubber disc and the plastic part, the rubber disc consisting of a convex surface, a concave backing corresponding to the convex surface, a hydrocolloid positioned in the concave backing, and a release film connected with the convex surface, a welding ring being disposed between the plastic part and the concave backing.

In the aforementioned convex stoma chassis, the size of the release film is larger than that of the hydrocolloid, and the size of the concave backing is consistent with that of the hydrocolloid.

In the aforementioned convex stoma chassis, a diameter of the release film is 3 mm larger than that of the hydrocolloid.

The aforementioned convex stoma chassis further comprises a label connected to the release film and printed with scales.

In the aforementioned convex stoma chassis, the release film is APET release film.

In the aforementioned convex stoma chassis, the backing is a PE foam backing.

In the aforementioned convex stoma chassis, an edge of the rubber disc is provided with a plurality of flanges.

In the aforementioned convex stoma chassis, a diameter of an inner ring of the plastic part is 50 mm.

In the aforementioned convex stoma chassis, a 12 mm pre-cut hole is defined at a middle position of the rubber disc.

The invention has the advantages that:
The design of the welding ring and an increase in a connectivity between the plastic part and the hydrocolloid chassis can completely isolate the contact of the excrement with the hot melt adhesive, and protect the hot melt adhesive from erosion, and improve the service reliability and service life.

The design of providing a plurality of flanges at the edge of the rubber disc facilitates the hydrocolloid to grasp the skin when adhering with convenience to peel off after use, and this symmetrical design is suitable for left-handed and right-handed users;

The design that the profile of the release film is larger than the diameter of the hydrocolloid can offset errors and hydrocolloid flow, and prevent the products from sticking during transportation and storage.

The diameter of the inner ring of the plastic part of the convex chassis is 50 mm, which can ensure that a force is applied at a proper position on the convex chassis (a pressure is applied at a position 1cm away from a stoma suture), and reduce the occurrence of complications.

### Brief Description of Drawings

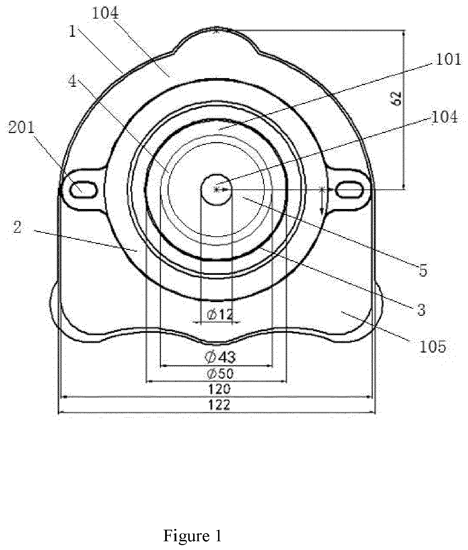

Fig. 1 is a main view of an embodiment of the present invention;

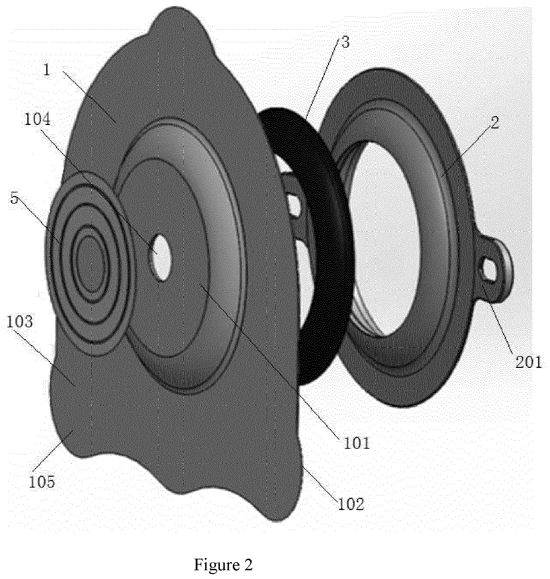

Fig. 2 is an exploded view of an embodiment of the present invention;

### Meaning of reference signs in the figure:

1 rubber disc, 101 convex surface, 102 concave backing, 103 release film, 104 pre-cut hole, 105 flange, 2 plastic part, 201 belt hole, 3 hot melt adhesive, 4 welding ring, 5 label.

### Detailed Description of Embodiments

The present invention will be described in detail below with reference to the drawings and specific embodiments.

A convex stoma chassis comprises a rubber disc 1, a plastic part 2 fixed on the rubber disc 1, and a hot melt adhesive 3 connected between the rubber disc 1 and the plastic part 2, the rubber disc 1 consisting of a convex surface 101, a concave backing 102 corresponding to the convex surface 101, a hydrocolloid positioned in the concave backing 102, and a release film 103 connected with the convex surface 101; and a front surface of the rubber disc 1 is with the release film 103, the back surface thereof is with the concave backing 102, and the middle is the hydrocolloid, and a welding ring 4 is disposed between the plastic member 2 and the concave backing 102. The design of the welding ring 4 and an increase in a connectivity between the plastic part 2 and the hydrocolloid chassis can completely isolate the contact of the excrement with the hot melt adhesive 3, and protect the hot melt adhesive 3 from erosion, and improve the service reliability and service life.

The width of the rubber disc 1 is 120 mm with a minimum area thereof meeting the use demand which is mainly an adhesive time, and the rubber disc 1 has a structure with a small upper part and a large lower part, and because the hydrocolloid liquid absorption mainly lies in the lower part, this design with the small upper part and large lower part makes full use of the hydrocolloid raw materials to increase the liquid absorption of the product. A bulge of the rubber disc 1 form the convex surface 101 and the concave backing 102 with a height of the bulge being 5 mm, which meets the requirements of most users, and it should be noted that the height of the bulge can vary according to the actual situation to meet the physical needs of patients. As a preference, the release film 103 is made of APET material, which has the following characteristics: it has a high heat resistance, which meets requirements of high temperature in a process, and generally can withstand a temperature up to 90°C; it has a good thermoplasticity, that is, it can keep its original properties after being molded and cooled; it has an appropriate tension and stiffness to meet requirements of the process. The backing of the rubber disc 1 is made of PE foam backing material, which has the following characteristics: it has a good heat sealability and can be thermally welded with the plastic part 2; it has a good bending modulus and can ensure that the rubber disc 1 changes along with a shape change of the skin when adhering to the skin; a back adhesive is provided on the back surface to ensure a good bonding with the hydrocolloid after compounding therewith. The edge of the rubber disc 1 is provided with a plurality of flanges 105, and the design of the plurality of flanges 105 facilitates the hydrocolloid to grasp the skin when adhering with convenience to peel off after use, and this symmetrical design is suitable for left-handed and right-handed users.

The size of the release film 103 is larger than that of the hydrocolloid, and the size of the concave backing 102 is consistent with that of the hydrocolloid; as a preference, the diameter of the release film 103 is 3 mm larger than that of the hydrocolloid; and such a design facilitates the peeling off because a starting point exists when the peeling off is made; because the hydrocolloid itself has a certain flexibility and fluidity, some errors and flow of the hydrocolloid can be appropriately offset if the size of the release film 103 is designed to be larger than that of the hydrocolloid, thus avoiding the adhesion of products during transportation and storage.

The convex stoma chassis further comprises a label 5 connected to the release film 103 and printed with scales. The label 5 is printed with scales which can be used to guide users to cut out an appropriate size, and the glue on the back of the label 5 is used to adhere to the release film 103 of the hydrocolloid, which can be made manually or by labeling equipment.

The diameter of the inner ring 2 of the plastic part of the convex disc is 50 mm. While that of most of the stoma are mainly more than 30 mm, the design of which can ensure that a force is applied at a proper position on the convex chassis(a pressure is applied at a position 1cm away from a stoma suture), and reduce the occurrence of complications. Special patients can also choose other specifications according to the actual situation. The plastic part 2 is made of a soft medical polymer material, which is friendly to the skin and has a heat welding performance at the same time to meet the heat welding with the ostomy bag film. Both sides of the plastic part 2 are provided with belt holes 201, which can be used together with an ostomy belt when needed to improve wearing reliability.

A 12 mm of pre-cut hole 104 is defined at the middle position of the rubber disc 1 to facilitate the use of ostomy scissors.

The invention provides a convex stoma chassis, in which a welding ring 4 is disposed between a plastic part 2 and a backing, so that the leakage of the excrement and the corrosion of the hot melt adhesive 3 can be completely avoided, and thus improving the use stability and the service life.

The above shows and describes the basic principles, main features and advantages of the present invention. The invention is defined in the following claims.

## Claims

1. A convex stoma chassis comprising a rubber disc (1), a plastic part (2) fixed on the rubber disc, and a hot melt adhesive (3) connected between the rubber disc and the plastic part; wherein the rubber disc comprises a convex surface (101), a concave backing (102) corresponding to the convex surface, a hydrocolloid positioned in the concave backing, and a release film (103) connected with the convex surface; a welding ring (4) is disposed between the plastic part and the concave backing.

2. The convex stoma chassis according to claim 1, wherein a size of the release film is larger than that of the hydrocolloid, and a size of the concave backing is consistent with that of the hydrocolloid.

3. The convex stoma chassis according to claim 2, wherein a diameter of the release film is 3 mm larger than a diameter of the hydrocolloid.

4. The convex stoma chassis according to claim 1, further comprising a label connected to the release film and printed with a scale.

5. The convex stoma chassis according to claim 1, wherein the release film is an APET release film.

6. The convex stoma chassis according to claim 1, wherein the backing is a PE foam backing.

7. The convex stoma chassis according to claim 1, wherein an edge of the rubber disc is provided with a plurality of flanges.

8. The convex stoma chassis according to claim 1, wherein a diameter of an inner ring of the plastic part is 50 mm.

9. The convex stoma chassis according to claim 1, wherein a 12 mm pre-cut hole is disposed at a middle position of the rubber disc.

## Patentansprüche

1. Konvexes Stoma-Chassis, das eine Gummischeibe (1), einen Kunststoffteil (2), der an der Gummischeibe befestigt ist, und einen Schmelzklebstoff (3) umfasst, der zwischen der Gummischeibe und dem Kunststoffteil verbunden ist; wobei die Gummischeibe eine konvexe Oberfläche (101), eine konkave Unterlage (102), die der konvexen Oberfläche entspricht, ein Hydrokolloid, das in der konkaven Unterlage positioniert ist, und einen Trennfilm (103) umfasst, der mit der konvexen Oberfläche verbunden ist; wobei ein Schweißring (4) zwischen dem Kunststoffteil und der konkaven Unterlage angeordnet ist.

2. Konvexes Stoma-Chassis nach Anspruch 1, wobei eine Größe des Trennfilms größer als die des Hydrokolloids ist und eine Größe der konkaven Unterlage mit der des Hydrokolloids übereinstimmt.

3. Konvexes Stoma-Chassis nach Anspruch 2, wobei ein Durchmesser des Trennfilms 3 mm größer ist als ein Durchmesser des Hydrokolloids.

4. Konvexes Stoma-Chassis nach Anspruch 1, das ferner ein Etikett umfasst, das mit dem Trennfilm verbunden und mit einer Skala bedruckt ist.

5. Konvexes Stoma-Chassis nach Anspruch 1, wobei der Trennfilm ein APET-Trennfilm ist.

6. Konvexes Stoma-Chassis nach Anspruch 1, wobei die Unterlage eine PE-Schaumunterlage ist.

7. Konvexes Stoma-Chassis nach Anspruch 1, wobei eine Kante der Gummischeibe mit mehreren Flanschen versehen ist.

8. Konvexes Stoma-Chassis nach Anspruch 1, wobei ein Durchmesser eines Innenrings des Kunststoffteils 50 mm beträgt.

9. Konvexes Stoma-Chassis nach Anspruch 1, wobei ein vorgeschnittenes 12 mm-Loch an einer mittleren Position der Gummischeibe angeordnet ist.

## Revendications

1. Châssis de stomie convexe comprenant un disque en caoutchouc (1), une pièce en plastique (2) fixée sur le disque en caoutchouc, et un adhésif thermofusible (3) connecté entre le disque en caoutchouc et la pièce en plastique ; le disque en caoutchouc comprenant une surface convexe (101), un support concave (102) correspondant à la surface convexe, un hydrocolloïde positionné dans le support concave, et un film détachable (103) connecté à la surface convexe ; une bague de soudage (4) étant disposée entre la pièce en matière plastique et le support concave.

2. Châssis de stomie convexe selon la revendication 1, une taille du film détachable étant plus grande que celle de l'hydrocolloïde, et une taille du support concave étant cohérente avec celle de l'hydrocolloïde.

3. Châssis de stomie convexe selon la revendication 2, un diamètre du film détachable étant 3 mm plus grand qu'un diamètre de l'hydrocolloïde.

4. Châssis de stomie convexe selon la revendication 1, comprenant en outre une étiquette connectée au film détachable et imprimée avec une échelle.

5. Châssis de stomie convexe selon la revendication 1, le film détachable étant un film détachable APET.

6. Châssis de stomie convexe selon la revendication 1, le support étant un support en mousse PE.

7. Châssis de stomie convexe selon la revendication 1, un bord du disque en caoutchouc étant pourvu d'une pluralité de brides.

8. Châssis de stomie convexe selon la revendication 1, un diamètre d'un anneau intérieur de la partie en plastique étant de 50 mm.

9. Châssis de stomie convexe selon la revendication 1, un trou prédécoupé de 12 mm étant disposé à une position médiane du disque en caoutchouc.
